# EUROPEAN PATENT APPLICATION

(11) **EP 3 858 397 A1**
(43) Date of publication of application: **04.08.2021**
(21) Application number: 20154827.8
(22) Date of filing: 31.01.2020
(51) Int. Cl.: A61M 1/10, A61M 1/12

(54) **INTRAVASCULAR BLOOD PUMP**

(71) Applicant: Abiomed Europe GmbH, 52074 Aachen (DE)
(72) Inventor: SPANIER, Gerd, 52074 Aaachen (DE); SIESS, Thorsten, 52074 Aachen (DE)
(74) Representative: Klunker IP Patentanwälte PartG mbB

(57) **Abstract**

An intravascular blood pump (1) comprises a pumping device and a catheter (5). The pumping device comprises a drive shaft (12), a rotor (10) located at a distal end of the drive shaft (5), a housing (11), in which the rotor (10) is housed, and a distal bearing (14) for rotatably supporting a distal end of the rotor (10). The distal bearing (14) comprises a static support member (18), which protrudes into or up against the distal end of the rotor (10). The rotor (10) and housing (11) may be expandable, and the pin (19) may have a sufficient length to prevent disengagement of the pin (19) from the distal end of the rotor (10) when the rotor (10) and the housing (11) are in a collapsed state.

## Description

### BACKGROUND OF THE INVENTION

This invention relates to an intravascular blood pump, in particular a percutaneously insertable blood pump, for supporting blood circulation in humans or optionally also in animals. For instance, the blood pump may be designed to be inserted percutaneously into a femoral artery of a patient and guided through the patient's vascular system in order, for example, to support or replace the pumping action of the heart.

While the present invention will be described in the context of an intravascular blood pump having an expandable housing, in which an expandable rotor is housed driven by an extracorporeal motor via a long and flexible drive shaft, the present invention is also applicable in other types of intravascular blood pumps, where the motor is located inside the patient's body next to the rotor and/or where the housing and rotor are not expandable.

A blood pump of the aforementioned expandable type is known, e.g., from US 2013/0303969 A1, which discloses a catheter pump assembly. An expandable housing is located at a distal end of the catheter. The expandable housing surrounds an expandable rotor driven by a flexible drive shaft, which extends through a first lumen of the catheter. The distal portion of the catheter pump assembly may be placed inside the heart via percutaneous access using the Seldinger technique, for example. The drive shaft contains a central lumen, which allows a guide wire together with its guide to be passed through the drive shaft to enable an exact positioning of the catheter pump assembly inside the heart. The rotor is rotatably supported in a bearing arranged at the end of the catheter and proximally of the rotor. The catheter comprises a second lumen for conveying a purge fluid in a distal direction to purge at least such bearing in order to prevent blood from entering into and clogging the bearing. Herein, "proximal" and "distal" are seen relative to the physician. Thus, proximal designates something which is relatively close to the physician whereas distal designates something which is relatively far away from the physician when the catheter is placed.

The document US 2013/0303970 A1 likewise describes an expandable catheter pump assembly, which comprises a proximal as well as a distal bearing. The rotor is mounted on the drive shaft between the proximal bearing and the distal bearing. The distal bearing is held in place by a stationary expandable distal bearing support, which is in slideable contact with the housing when it is in its expanded state. The distal bearing support comprises a self-sealing septum, which allows the guide wire and its guide to pass through. When the guide wire and its guide are removed from the catheter pump assembly, the septum reseals and, thus, prevents blood from entering into the drive shaft.

One advantage of the distal bearing is that the gap between the rotor blades and the internal surface of the housing can be better controlled to avoid blood damage even when rotors with large diameters are employed. In other arrangements, the distal end of the drive shaft is mounted in a distal bearing arranged at the distal end of the housing. However, intravascular blood pumps with a drive shaft supported in a distal bearing distally from the rotor have the problem that tendinous structures of the heart can be drawn into the housing and become entangled around the drive shaft. This can lead to the destruction of heart structures as well as damage to the intravascular blood pump and an increased risk of blood clots forming at the distal bearing.

To avoid entanglement of tissue with rotating parts, EP 2047873 A1 describes polyurethane drive shaft covers and bearing-hub constellations that separate the rotating shaft from the blood. But there usually remains a gap and a rotating part of the hub, such that the rotating shaft is exposed to its surroundings. This is problematic as tendinous structures of the heart may be caught in that gap or on the distal part of the hub, which may lead to injury of the patient and damage to the intravascular blood pump.

Accordingly, there is a need for distally supporting the rotor inside the housing of an intravascular blood pump without the danger of tendinous structures of the heart becoming entangled in the pump.

### SUMMARY OF THE INVENTION

According to a first aspect of the invention, an intravascular blood pump comprises a pumping device and a catheter. The pumping device comprises a drive shaft, a rotor located at a distal end of the drive shaft and housed in a housing, and at least a distal bearing for rotatably supporting a distal end of the rotor. Furthermore, in the blood pump disclosed herein, the distal bearing comprises a static support member which protrudes into or up against the distal end of the rotor.

Accordingly, the drive shaft is not supported in the distal bearing but instead the rotor is mounted to the very end of the drive shaft so that it is the distal end of the rotor, which is supported by a static support member extending into or up against the rotor. In this way, tendinous structures are less likely to be caught by rotating parts, in particular if no rotating cylindrical structure extends beyond the leading edge of the rotating blade. This leads to a safer intravascular blood pump with a longer lifetime.

In embodiments in which the drive shaft is driven by an extracorporeal electric motor, the drive shaft preferably extends from a proximal end region of the catheter to a distal end region of the catheter. The drive shaft is typically flexible and preferably hollow. The drive shaft preferably consists of or comprises a flexible cable which is preferably formed of differently oriented fiber layers. In particular, the drive shaft cable is most preferably made up of a plurality of coaxial windings, preferably with different winding directions, particularly preferably with alternating winding directions, running spirally around a lumen extending axially along the drive shaft. For example, a drive shaft cable can comprise two coaxial windings, with opposite winding directions, and an outer diameter of the drive shaft cable may be between 0.4 mm and 2 mm, preferably between 0.6 mm and 1.2 mm, particularly preferably between 0.8 mm and 1.0 mm. The proximal end of the drive shaft cable is preferably attached to the extracorporeal electric motor. The drive shaft cable serves to transfer a torque from the electric motor to the rotor at the distal end of the drive shaft. In some cases, the drive shaft cable may comprise a stiff, rigid shaft at its distal end, onto which the rotor is attached inside the housing, in order to provide stability to the rotor.

In the distal end region, the drive shaft is in some embodiments reinforced by a reinforcement element, for example a metal wire or a carbon wire, that is provided in the lumen extending axially along the drive shaft. In one embodiment the metal wire is made of 1.4310 stainless steel.

The intravascular blood pump is preferably designed as an expandable blood pump with a housing having an expandable section. In some embodiments, the housing comprises or consists of a shape-memory material, in particular Nitinol. The diameter of the percutaneously insertable blood pump is generally limited by the internal diameter of the smallest blood vessel to be traversed. The intravascular blood pump may be moved through blood vessels with the housing in its collapsed state. On reaching the heart or larger vessels, the housing of the intravascular blood pump may be expanded. This allows the percutaneous insertion of a larger blood pump into the heart than otherwise possible. With such a larger blood pump, it may be possible to generate larger blood flow rates.

When the blood pump is designed as an expandable pump, a cannula is preferably provided around a portion of the drive shaft, which lies in the vicinity of the rotor, and the housing and the rotor are configured to be transferred at least in part into the cannula. During such a transfer, the expandable section of the housing and the rotor are compressed at least along a radial direction extending transversely to a longitudinal direction, from an expanded state to a compressed state. Preferably, parts of the rotor, such as the rotor blades, or the entire rotor, are also expandable to allow a larger rotor to be inserted into the heart, which may improve flow rates.

In some embodiments, the static support member protrudes up against the distal end of the rotor. In comparison to embodiments in which the static support member protrudes into the rotor, a particularly flexible pump section of the intravascular blood pump may be created. High flexibility of the pumping device is specifically advantageous during insertion and removal of the intravascular blood pump. If the static support member does not protrude into the rotor and is instead merely placed up against the distal end of the rotor, it may intentionally dislodge from the rotor when the pump section is bent during maneuvering of the pumping device through the blood vessels. When the pump section reaches its final destination inside the heart, it may straighten and the static support member may resume a position in which it protrudes up against the distal end of the rotor.

Preferably, the static support member is attached to the distal end of the housing, wherein the expansion of the housing may provide an axial force via the static support member onto the distal end of the rotor. Preferably, the force is equal to or less than 1.8 N. When the static support member protrudes up against the distal end of the rotor, it may limit further expansion of the housing.

When the housing is compressed, the static support member preferably moves away from the distal end of the rotor. In this state, the pump section is more flexible as relative radial movement of the static support member and the rotor becomes possible. This may be advantageous during insertion of the intravascular blood pump or during retrieval.

In certain embodiments, the intravascular blood pump comprises a nose at the distal end of the rotor. When the housing is in its expanded state, the nose protrudes into the static support member, which preferably possesses a correspondingly formed recess. The nose has the purpose to center the rotation of the rotor and to bring the rotor and the static support member into a correct relative position after expansion of the housing. The nose preferably protrudes over the surrounding surface of the rotor by between 0.1 mm and 2 mm, more preferably between 0.2 mm and 1 mm and most preferably between 0.3 mm and 0.5 mm. The depth of the recess in the static support member corresponds to the nose and is preferably between 0.1 mm and 2 mm, more preferably between 0.2 mm and 1 mm and most preferably between 0.3 mm and 0.5 mm.

In some embodiments in which the static support member protrudes into the rotor, the rotor comprises an axial stop for the static support member, such as a recess at its distal end having a bottom or a step. The bottom or step defines an axial stop for a proximal end of the static support member in the distal end of the rotor. This is particularly advantageous in the context of an expandable blood pump. In its expanded state, the proximal end of the static support member, which protrudes axially into the rotor, may be in contact with the axial stop, thereby preventing further expansion of the housing and, thus, limiting a radial gap width between an outer edge of the rotor blades and an inner surface of the expandable housing. Alternatively, in the expanded state of the expandable blood pump, the proximal end of the static support member and the axial stop may form a gap, which is preferably between 0.01 mm and 1 mm, more preferably between 0.01 mm and 0.1 mm and most preferably between 0.01 mm and 0.05 mm wide in an axial direction.

A length of the recess at the distal end of the rotor, which is measured in an axial direction, may for instance be between 0.5 mm and 8 mm, preferably between 1 mm and 5 mm, particularly preferably between 1.5 mm and 2.5 mm. When the housing is moved into the cannula, the housing preferably stretches by between 0.5 mm and 2.5 mm axially, more preferably between 1 mm and 2 mm, most preferably by approximately 1.7 mm.

Inside the distal end of the drive shaft, i.e., inside the rotor shaft, the intravascular blood pump may contain an optional fluid line arranged to guide a purge fluid through the rotor to the distal bearing. In some embodiments, the rotor comprises a hollow section as a part of the fluid line, wherein the intravascular blood pump is arranged to guide the purge fluid through the hollow section of the rotor to the distal bearing. The purge fluid may be transported to the fluid line via the catheter. In cases in which the drive shaft extends through the catheter and is driven by an extracorporeal electric motor, the purge fluid may enter the catheter and/or the drive shaft inside a housing of the electric motor. The purge fluid may flow inside the catheter adjacent to the drive shaft. Where the drive shaft is hollow, purge fluid may flow partly, predominantly or entirely through the drive shaft lumen. From the distal end of the catheter to the rotor, the purge fluid may flow through the drive shaft. At least in the space between the distal end of the catheter and the proximal end of the rotor, the drive shaft may comprise a cover to avoid the purge fluid from leaking from said space.

Alternatively, the purge fluid may not be guided through the main lumen of the catheter, which contains the drive shaft, but through one or more separate, secondary lumina. In cases in which the electric motor is placed inside the patient's body along with the pump section, the purge fluid may equally flow through the catheter towards said fluid line.

At the distal end region of the catheter, the purge fluid preferably transfers into the fluid line inside the rotor shaft. In some cases, the rotor shaft or the rotor hub may have a central lumen to accommodate the fluid line. In particular, in the case of a hollow drive shaft cable, the drive shaft cable may extend into the rotor to form both the rotor shaft and the fluid line, or the hollow drive shaft cable may be extended by a hollow tube to form both the rotor shaft and the fluid line. The hollow drive shaft cable may be permeable to purge fluid.

In a purged distal bearing, blood is less likely to enter the bearing gap. As a result, blood clots are prevented. In addition, a purged bearing may have less friction than the alternative distal bearings in the prior art. In particular, the purge fluid lubricates the bearing and can transport frictional heat away from the bearing. This may allow higher rotational speeds, lower power consumption and an increased lifetime of the blood pump. The purge fluid may be any biocompatible fluid suitable for purging the distal bearing. Examples of a suitable medical fluid include saline solution with or without heparin, glucose solution and/or water.

In alternative embodiments, the distal bearing is not purged. Accordingly, there is no transport of purge fluid to the distal bearing and the intravascular blood pump may not comprise a fluid line.

The distal bearing is preferably arranged such that the purge fluid may exit between the static support member and the distal end of the rotor, into which or up against which the static support member protrudes. Preferably, the distal bearing is arranged such that the purge fluid flows from a distal end of the fluid line to the distal bearing. In particular, the intravascular blood pump may be arranged such that any purge fluid passing through the hollow drive shaft or rotor shaft exits entirely or at least in part through the distal bearing. By applying a suitable pressure, the purge fluid may be urged through the bearing gap of the distal bearing, which is the gap bounded by the static support member and the adjacent section of the rotor. Preferably, the pressure of the purge fluid is in a range of 300 mmHg (0.4 bar) to 1500 mmHg (2 bar), more preferably in a range of 600 mmHg (0.8 bar) to 1100 mmHg (ca. 1.5 bar). If the distal bearing is purged and the rotor comprises a nose that protrudes into the static support member, the nose may contain at least one opening to allow the purge fluid to enter the bearing gap between the nose and the static support member.

In some embodiments, a distal end of the static support member is mounted at a distal end of the housing. The distal end of the housing may provide stable support for the static support member which supports the distal end of the rotor.

The static support member preferably comprises a pin extending from distally to proximally and protruding up against or, preferably, into the distal end of the rotor. Thus, the pin may be arranged to form the distal bearing for the rotor. In embodiments in which the distal bearing is purged, the pin is preferably arranged such that purge fluid may exit between the pin and the rotor mounted on the pin.

Preferably, the pin possesses a circular cross-section. However, other cross-sections are equally possible in the distal part of the pin which is located outside the rotor. For example, the pin may have an oval cross-section. In some embodiments, the pin may be hollow. Alternatively, the pin may be made of solid material. Preferably, the pin is tapered towards its proximal end. The pin may be elastically bendable, preferably such that during bending of the pump head, the rotor keeps concentric to the housing.

Preferably, an inner diameter at the distal end of the rotor, into which the static support member, in particular the pin, axially protrudes, is between 0.3 mm and 1.5 mm, more preferably between 0.5 mm and 1.2 mm and most preferably between 0.7 mm and 0.9 mm wide. Preferably, the radial bearing gap between the outside of the pin and its opposite bearing surface is between 1 µm and 10 µm, more preferably between 2 µm and 8 µm wide.

In some embodiments, the pin is particularly long and protrudes into the rotor and extends proximally through the entire rotor. Preferably, the pin exits the rotor proximally and continues inside the drive shaft, e.g., ending inside the proximal bearing. In this case, the end of the pin may be arranged inside the portion of the drive shaft that is situated in the proximal bearing. By employing such a long pin that extends through the entire length of the rotor and into the proximal bearing, a particularly stiff and low-vibration pump may be created. Alternatively, the pin may extend even further to a point proximally of the proximal bearing. The pin extending through the rotor may be purged or unpurged and may be used in conjunction with a hollow drive shaft, with a drive shaft that is not hollow or that is only hollow along a part of its length.

Preferably, the material of the pin comprises at least one of the following materials: A biocompatible material, in particular one or more of MP35N, 35NLT, Nitinol, stainless steel (in particular medical-grade stainless steel) and ceramics. The surface of the pin may comprise a coating, for example a diamond-like carbon (DLC) coating.

Preferably, the length of the pin by which the pin protrudes into the distal end of the rotor during an operational state of the intravascular blood pump is between 0.5 mm and 8 mm, preferably between 1 mm and 5 mm, particularly preferably between 1.5 mm and 2.5 mm. The longer the internal length is, the stiffer the rotor is and, thus, the better controllable is the width of the gap between the outer edge of the rotor blades and the inner surface of the housing. The blades must not touch the inner surface of the housing, and the gap should be sufficiently large to prevent blood damage. A stiffer rotor can also be operated with lower excursions and less vibration, which improves hemocompatibility.

The pin may have a sufficient length to remain within the distal end of the rotor when the housing and the rotor are in the compressed state. The length of the pin, which remains inside the distal end of the rotor when the housing and the rotor are in the compressed state, is preferably more than 1.5 mm, more preferably more than 1.7 mm and most preferably more than 2 mm. When the housing and the rotor are compressed before deployment of the blood pump, the housing is extended in a longitudinal direction and the static support member, in particular the pin, extending into the distal end of the housing may possibly move out of the rotor entirely. Then, when the housing is expanded again, the pin might not move back into the rotor and the pump may not be functional. Therefore, if the pin is chosen with a sufficient length such that the pin stays inside the rotor even in the compressed state of the housing, such a problem may be avoided.

In embodiments with a pin, the distal bearing surfaces are the surface of the pin as well as a distal outer bearing surface, which may be provided by the rotor itself or by a distal bearing sleeve in the hub of the rotor. In some cases, the distal outer bearing surface may be provided by the stiffening element mentioned above.

The distal bearing sleeve may have an inner diameter preferably ranging from 0.3 mm to 1.5 mm, more preferably from 0.5 mm to 1.2 mm and most preferably from 0.7 mm to 0.9 mm.

In some embodiments, the intravascular blood pump comprises a flexible atraumatic tip to avoid damage to the patient's tissue. The atraumatic tip can be made of a flexible medical-grade polymer such as Pebax® or Polyurethane. Preferably, the flexible atraumatic tip is designed as a pigtail or in a J-form.

Preferably, the intravascular blood pump comprises a proximal bearing in addition to the distal bearing. The proximal bearing may be located inside a distal end region of the catheter or a proximal end region of the housing. If the proximal bearing is purged, purge fluid may exit the catheter via the bearing gap of the proximal bearing. The bearing gap of the proximal bearing is preferably between 1 µm and 10 µm, more preferably between 2 µm and 8 µm.

According to a second aspect of the invention, the intravascular blood pump described above is used in a patient, that is, it is inserted and operated inside the patient to support blood flow. In particular, a purge fluid may be supplied to the intravascular blood pump and exit via the fluid line through the distal bearing.

### BRIEF DESCRIPTION OF THE DRAWINGS

Hereinafter the invention will be explained by way of example with reference to the accompanying drawings. The accompanying drawings are not drawn to scale. In the drawings, each identical or nearly identical component that is illustrated in various figures is represented by the same numeral. For purposes of clarity, not every component may be labeled in every drawing. In the drawings:
Fig. 1 is a schematic representation of an intravascular blood pump, which is positioned within the left ventricle of the heart;
Fig. 2 shows a schematic representation of an intravascular blood pump;
Figs. 3A and 3B show schematic representations of an intravascular blood pump in an expanded and a compressed state;
Figs. 4A, 4B and 4C show a schematic representation of an intravascular blood pump with a static support member extending into the distal end of the rotor according to a first embodiment;
Fig. 5 shows a schematic representation of an intravascular blood pump with a static support member extending into the distal end of the rotor according to a second embodiment;
Figs. 6A to D show a schematic representation of an intravascular blood pump with a rotor having a nose at its distal end according to a third embodiment;
Fig. 7 shows a schematic representation of an intravascular blood pump with a proximal and a distal bearing; and
Figs. 8A and 8B show a schematic representation of the path of purge fluid in an intravascular blood pump.

### DETAILED DESCRIPTION OF THE DRAWINGS

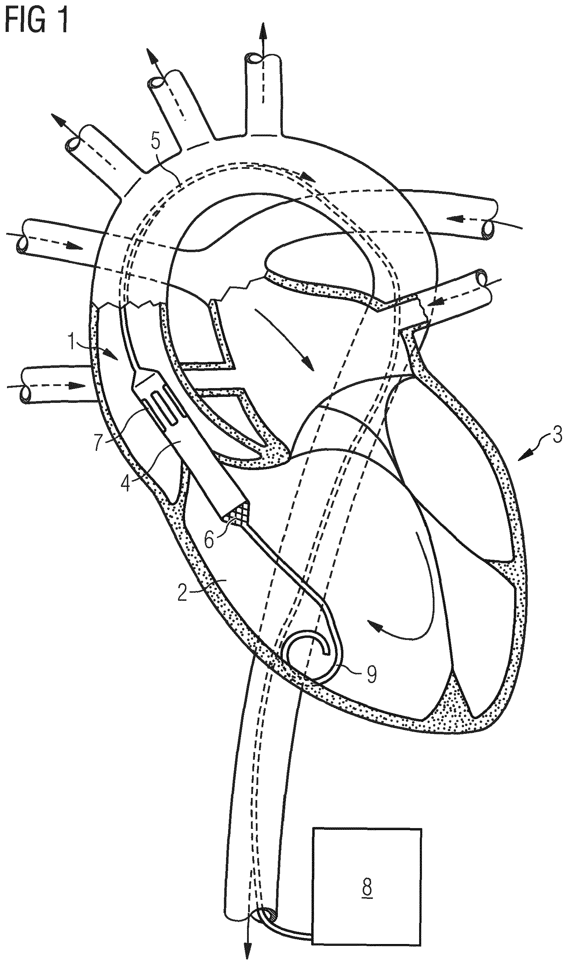

Fig. 1 shows the use of an intravascular blood pump 1 for supporting, in this particular example, a left ventricle 2 of a human heart. The intravascular blood pump 1 comprises a catheter 5 and a pumping device, the pumping device comprising a pump section 4 mounted at a distal end region of the catheter 5. The intravascular blood pump 1 may be placed inside the heart using a percutaneous, transluminal technique. For example, the intravascular blood pump 1 may be introduced through a femoral artery. However, alternative vascular access is equally possible, such as access through the subclavian artery. After passing through the femoral artery, the catheter 5 may be pushed into the aorta such that the pump section 4 reaches through the aortic valve into the heart. The positioning of the pump section 4 in Fig. 1 serves purely as an example, whereas different placements are possible, such as positioning the pump section 4 inside the right ventricle of the heart.

The pump section 4 comprises a rotor 10 to cause blood to flow from a blood flow inlet 6 at a distal end of the pump section 4 to a blood flow outlet 7 located proximally of the blood flow inlet 6. The catheter 5 houses a drive shaft 12 driven by the electric motor 8, which is preferably placed outside the patient's body. The drive shaft 12 drives the rotor contained inside the pump section 4. At its distal end, the pump section 4 possesses a flexible atraumatic tip 9 having the form of a pigtail or a J-form, which facilitates placement of the intravascular blood pump 1 by aiding navigation inside the patient's vascular system. Furthermore, the softness of the flexible atraumatic tip 9 allows the pump section 4 to support itself atraumatically against the wall of the left ventricle 2.

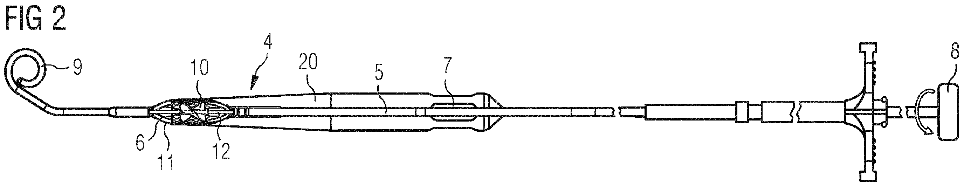

Fig. 2 shows the intravascular blood pump 1 in further detail. The rotor 10 is located inside a housing 11. In this embodiment, both the rotor 10 and the housing 11 are compressible. In this case, the intravascular blood pump 1 is transported through the patient's vascular system while both the rotor 10 and the housing 11 are in their compressed state. Once the pump section 4 is at its target location, the housing 11 and rotor 10 are expanded. The flexible atraumatic tip 9 is situated at the distal end of the housing 11. The drive shaft 12 is realized as a flexible drive shaft cable. The drive shaft 12 with the rotor 10 arranged at a distal end thereof can be seen protruding from the distal end of the catheter 5. When the rotor 10 inside the housing 11 is rotated by means of the drive shaft 12, blood is drawn into the blood flow inlet 6 at the distal end of the housing 11 and through the housing 11 into a downstream tubing 20, which is attached to the housing 11 and extends proximally. The blood is then ejected from the downstream tubing 20 into the aorta through the more proximally located blood flow outlet 7 provided in the downstream tubing 20. The downstream tubing 20 is made of a flexible material such that it can be compressed by the aortic valve as the patient's heart is pumping. The downstream tubing 20 is typically expanded mainly due to the active blood flow generated by the rotor 10 during rotation. By placing the blood flow inlet 6 inside the left ventricle 2 and the blood flow outlet 7 inside the aorta, the intravascular blood pump 1 may support the patient's systemic blood circulation. If the intravascular blood pump 1 is configured and placed differently, it may be used, e.g., to support the patient's pulmonary blood circulation instead.

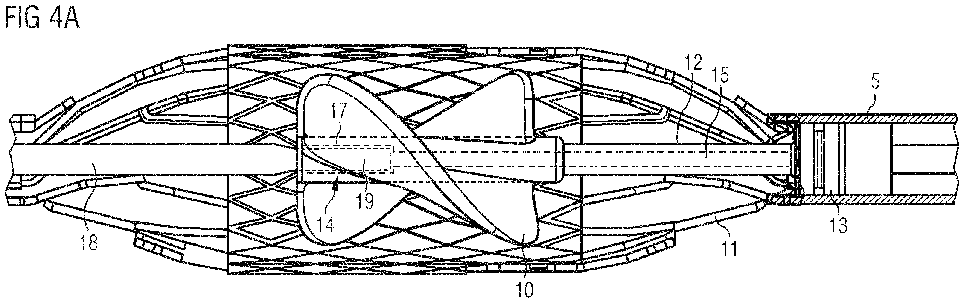

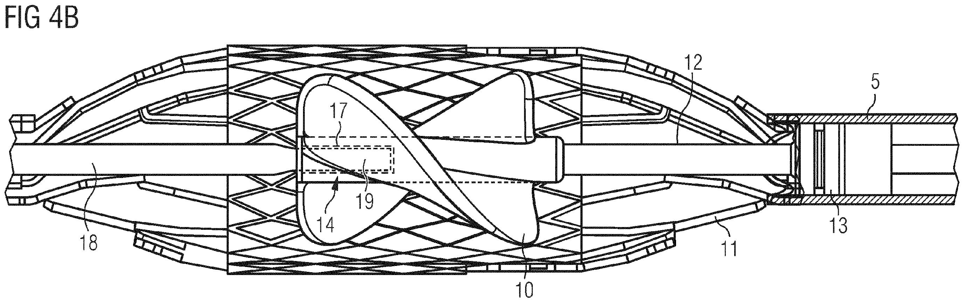

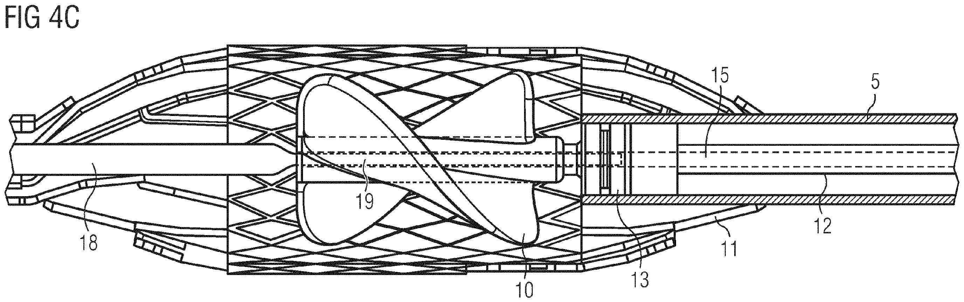

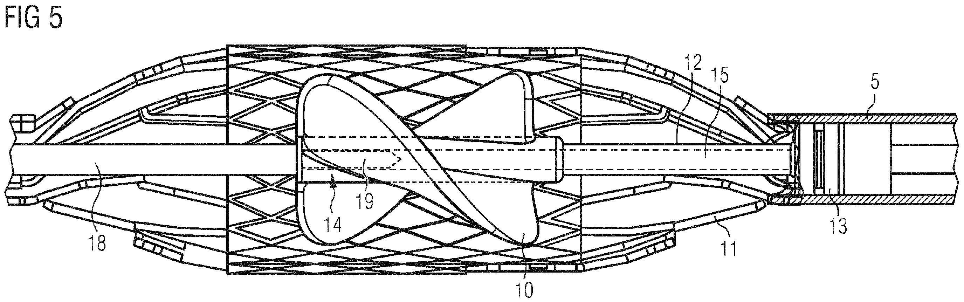

In this example, a liquid, in particular a purge fluid, is supplied from outside the patient's body through the catheter 5 to the pump section 4. Inside the pump section 4, the liquid may be used to purge one or more bearings in order to reduce friction and cool the pump section 4, as will be explained further in relation to Figs. 4 and 5. Preferably, the liquid is used to purge at least the distal bearing. In such case, the pressure of the purge fluid is chosen to be higher than the blood pressure of the patient in order to prevent blood from entering the bearing. Preferably, the pressure of the purge fluid is in a range of 300 mmHg (0.4 bar) to 1500 mmHg (2 bar), more preferably in a range of 600 mmHg (0.8 bar) to 1100 mmHg (ca. 1.5 bar).

The housing 11 is preferably produced from a shape-memory material, such as Nitinol, and provides a cage around the rotor 10. As can be seen in Fig. 5, a central part of the housing 11 carries a sleeve, which defines a channel through which blood is pumped by means of the rotor 10. Proximally and distally of this channel, the housing 11 allows blood to be sucked into the housing 11 and pushed out of the housing 11 into the downstream tubing 20 (as shown in Fig. 2).

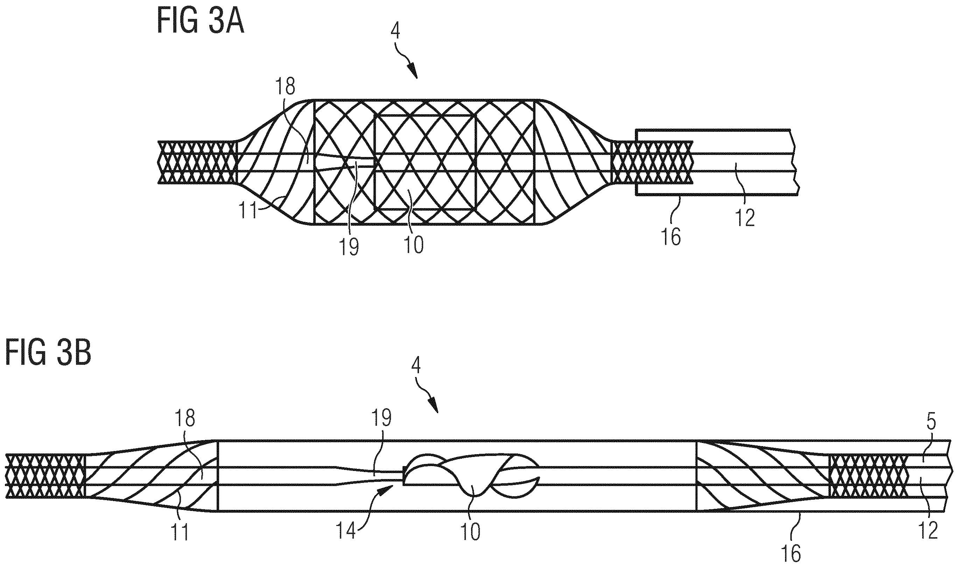

Figs. 3A and 3B show the pump section 4, its rotor 10 as well as its housing 11 in an expanded and in a compressed state, respectively. A cannula 16 is arranged at the distal end of the catheter 5. Initially, before deployment of the intravascular blood pump 1, the pump section 4 is provided in its compressed state inside the cannula 16. The cannula 16 can be a cannula 16 pertaining to the catheter 5 or a peel-away-sheath to aid the insertion of the catheter 5 into the body of a patient. When a physician has determined that the catheter 5 is placed correctly inside a patient's vascular system, he or she will push the housing 11 out of the cannula 16. With the cannula 16 removed, the housing 11 will expand due to its shape-memory properties. At the same time, the rotor 10 expands due to its elasticity. As the housing 11 expands radially away from the drive shaft 12, it contracts in the longitudinal direction.

The rotor 10 is supported in a distal section of the rotor 10 by a distal bearing 14 comprising a static support member 18 with a pin 19, the static support member 18 being attached to the housing 11 at one end thereof and extending into the distal end of the rotor 10 with its pin 19 on the other end thereof so that upon the expansion of the housing 11 the pin 19 can move axially inside the distal end of the rotor 10. Preferably, the pin 19 is sufficiently long for it to remain inside the rotor 10 when the housing 11 is in its compressed state. When the intravascular blood pump 1 is in its expanded state and needs to be removed from the heart, the physician pulls the housing 11 back into the cannula 16, which will cause the housing 11 to compress radially and extend longitudinally so that the distal end of the housing 11 moves away from the rotor 10 along with the static support member 18 and its pin 19, which extends into the distal end of the rotor 10. The smaller diameter of the housing 11 thus achieved facilitates the removal of the intravascular blood pump 1 from the patient.

In prior art distal bearings 14, the drive shaft 12 sometimes extends distally of the rotor 10 into the bearing. This, however, may cause tendinous chords of the heart to be entangled with the drive shaft 12 possibly leading to clotting and device failure. Therefore, the use of the static support member 18 as part of the distal bearing 14, which does not involve rotating parts distal to the rotor 10 and distal to the rotor blades, is advantageous.

Figs. 4A and 4B show the pump section 4 according to a first embodiment in further detail including the housing 11 and the rotor 10, which is driven by the drive shaft 12. The drive shaft 12 is rotatably supported both in a proximal bearing 13 at the distal end of the catheter 5 proximally of the rotor 10 (or in a proximal part of the housing) and in a distal bearing 14 located at the distal end of the rotor 10. In Fig. 4A, the drive shaft 12 is hollow at its distal end or, more specifically, the rotor shaft is hollow so as to form a fluid line 15 through which a purge fluid may be pumped towards the distal bearing 14. Where the drive shaft is hollow and extends up to the distal end of the rotor 10, the rotor 10 may be formed directly on the distal end of the drive shaft 12 so that the rotor shaft is formed by the drive shaft, whereby in the regions of the proximal and distal bearings the drive shaft 12 may be stiffened, e.g., by injection molded plastic material, and provided with appropriate outer and inner bearing surface finishes, respectively. Alternatively, the entire end region including the bearing sections of the drive shaft 12 may be stiffened in order to obtain a stiffer structure of the pump section. For example, a stiff hollow tube is slipped over the end of the drive shaft 12 and extends distally to form the rotor shaft and bearing sections. The purge fluid may be transported through the fluid line 15 in the rotor shaft to the distal bearing 14. In the embodiment shown in Fig. 4A, the purge fluid can be urged through the central fluid line 15 to exit the drive shaft 12 at its distal end and further through a bearing gap of the distal bearing 14 into the blood stream. The purging of the distal bearing 14 by the purge fluid leads to less friction and thus to less wear on the distal bearing and, furthermore, prevents blood from entering into and clogging the bearing gap.

For the intravascular blood pump 1 to be efficient, a large rotor 10 diameter is desirable. However, as the gap between the rotor 10 and the housing 11 gets smaller, the risk of blood cells or the rotor 10 being damaged increases. If only a proximal bearing 13 is used, the system may oscillate and the gap between the tip ends of the blades of the rotor 10 and the inner surface of the housing 11 may undergo large variations. When the flexible atraumatic tip 9 touches the heart wall, the movement of the heart can cause bending of the housing, which could lead the housing to touch the rotor. Touching of housing and rotor during use could cause a significant increase of damage to blood cells. By using both a proximal bearing 13 and a distal bearing 14, as illustrated in Figs. 4A and 4B, the position of the rotor 10 is more stable and the variation of the size of said gap is lower than with just one bearing. For a given housing 11, this may allow the rotor 10 diameter to be larger, which allows for a higher flow rate of the intravascular blood pump 1 without the housing touching the rotor.

At its distal end, the rotor 10 comprises a recess 17. The static support member 18 fixed relative to the distal end of the housing 11 protrudes with its pin 19 into the recess 17. The bottom 19 of the recess 17 in Fig. 4A is formed as a step and defines a stop inside the rotor 10 against which the pin 19 of the static support member 18 can rest. In Fig. 4A, the fluid line 15 penetrates the bottom of the recess 17 to allow purge fluid to exit the distal bearing 14 between the pin 19 and the recess 17.

The embodiment of the intravascular blood pump 1 in Fig. 4B is similar to the embodiment in Fig. 4A. Importantly, however, the distal bearing in Fig. 4B is not purged and is designed to operate in blood instead. Thus, the drive shaft 12 does not need to be hollow. Accordingly, there is no fluid line 15 in Fig. 4B. The bottom of the recess 17 does not contain an opening for purge fluid to flow through the bearing gap between the pin 19 and the recess 17. In such an embodiment, less purge fluid may be required. If the proximal bearing is not purged, the intravascular blood pump may require no purge fluid at all.

Fig. 4C shows a similar embodiment to Figs. 4A and 4B. Here, the pin 19 is particularly long and extends proximally through the rotor shaft and into the drive shaft 12. In the embodiment of Fig. 4C, the proximal end of the pin 19 is located inside the part of the drive shaft 12, which is located inside the proximal bearing 13. In alternative embodiments, the proximal end of the pin 19 may be located, e.g., proximally of the proximal bearing 13 or between rotor and proximal bearing.

By having the pin 19 extend into the proximal bearing 13, a greater stiffness of the intravascular blood pump 1 may be achieved. Furthermore, the pin 19 shown in Fig. 4C may help to reduce vibrations of the intravascular blood pump 1 during its operation and may decrease undesired bending.

The proximal bearing 13 in Fig. 4C is located inside the housing 11, distally of the proximal bearing's 13 location in Figs. 4A and 4B. The distance between the proximal bearing 13 and the rotor 10 is particularly small in the embodiment shown, e.g., smaller than the outer diameter of the proximal bearing 13. The short distance may further increase the stiffness of the intravascular blood pump 1.

The pin 19 in Fig. 4C is combined with a hollow drive shaft 12 such that, in some embodiments, purge fluid may flow through the drive shaft 12 and past the pin 19 to exit at the distal end of the rotor 10. Alternatively, no purge fluid may be used in some embodiments. In this case, the long pin 19 of Fig. 4C may be combined with a drive shaft that is not hollow or only hollow along some part of its length.

Fig. 5 shows the pump section 4 according to a second embodiment again with a compressible housing 11 and a rotor 10 driven by a hollow drive shaft 12, which is rotatably supported in a proximal bearing 13 arranged proximally of the rotor 10 at the distal end of the catheter 5. In this embodiment, the pin 19 of the static support member 18 forming part of the distal bearing

14 has a pointed end. If the dimensions of the housing 11 and the pin 19 are such that the pin 19 leaves the rotor 10 when the housing 11 is compressed, the pointed end of the pin 19 facilitates reintroduction of the pin 19 into the opening at the distal end of the rotor 10 when the housing 11 is expanded again. Preferably, the pin 19 is sufficiently long for the pin 19 to remain inside the rotor 10 when the housing 11 is in the compressed state. This may avoid the circumstance, in which the pin 19 fails to re-enter the rotor 10 when the housing 11 is being expanded. In some cases, it is not necessary for proper function that a required bearing gap is present over the full length of the pin 19. Rather, it is sufficient for the bearing gap between the outside of the pin 19 and its opposite bearing surface to be between 1 µm and 10 µm, more preferably between 2 µm and 8 µm wide in at least one location.

In this embodiment, rather than providing a bottom or a step in the opening at the distal end of the rotor 10, the static support member 18 may be provided with a shoulder against which the rotor 10 abuts in an expanded state of the housing 11, thereby limiting further expansion of the housing 11, if desired. In some embodiments, the distal bearing 14 may exclusively be a radial bearing.

Again, a purge fluid may be supplied through the fluid line 15 of the drive shaft 12 towards a distal bearing 14, pass by the pin 19, which forms a distal radial bearing for the rotor 10, and leave the rotor 10 at its distal end. This prevents blood from entering the rotor 10, reduces friction and cools the distal bearing 14. Alternatively, the distal bearing 14 may not be purged. Accordingly, there may not be a fluid line 15.

Furthermore, in the embodiment shown in Fig. 5, the pin 19 sits inside the central duct 15 of the rotor 10 when the housing 11 is expanded. In this case, for example, the drive shaft 12 may terminate at the distal end surface of the rotor 10. Alternatively, the distal end of the drive shaft 12 may be located inside the rotor 10, e.g., at the level of the bottom of the recess as seen in the embodiment of Fig. 4A so as to form the stop for the pin 19.

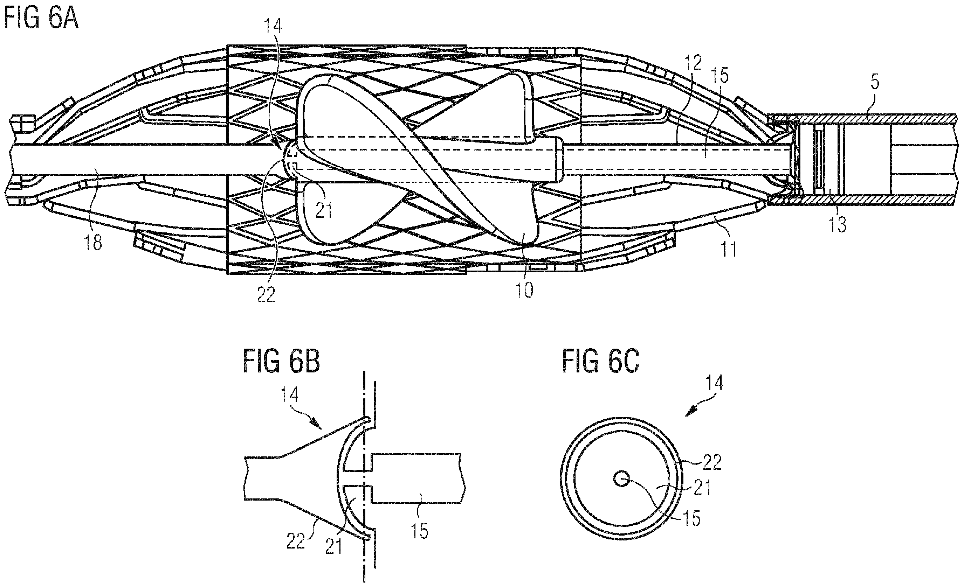

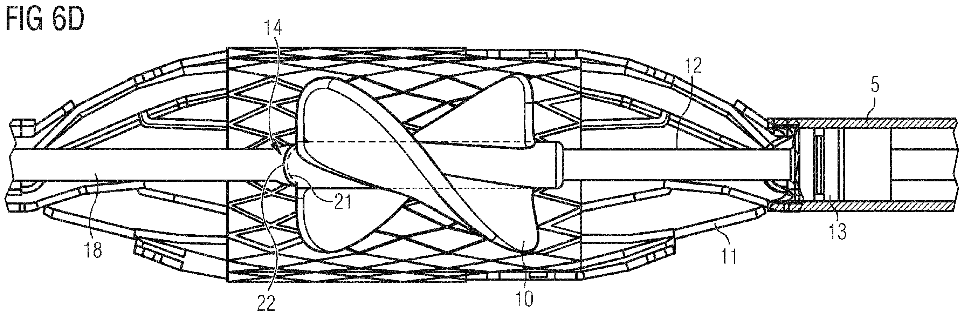

Figs. 6A, 6B, 6C and 6D show a third embodiment of the pump section 4 with the compressible housing 11 and the static support member 18, which is attached to the housing 11. The rotor 10 comprises a nose 21 at its distal end. In Figs. 6A, 6B and 6C, the fluid line 15 inside the distal end of the drive shaft 12 leads to an opening in the nose 21 through which purge fluid may enter the bearing gap of the distal bearing 14 between the nose 21 and a corresponding recess 22 at the proximal end of the static support member 18. In Fig. 6D, however, the distal bearing 14 is unpurged. Thus, the embodiment in Fig. 6D does not possess a fluid line 15 and an opening in the nose 21. The unpurged distal bearing 14 may reduce the amount of purge fluid needed to operate the intravascular blood pump 1. In combination with an unpurged proximal bearing 13, the intravascular blood pump 1 may need no purge fluid at all.

When the housing 11 is compressed, the nose 21 dislodges from the recess 22 and thus the intravascular blood pump 1 becomes more flexible. When the housing 11 is expanded at the target site, the nose 21 automatically moves into the recess 22, wherein the conical or spherical shape of the nose 21 helps to guide the nose 21 into the recess 22 and centers the rotor 10 with respect to the static support member 18. Fig. 6B shows an enlarged section of the distal bearing 14 with the nose 21 at the rotor 10 and the corresponding recess 22. A vertical dashed line in Fig. 6B shows the cross-sectional plane of Fig. 6C. The cross-section exhibited in Fig. 6C displays the distal bearing 14 in concentric circles. From periphery to center, the concentric circles show the recess 22, the distal bearing gap between recess 22 and nose 21, the nose 21 and the opening of the fluid line 15 into the distal bearing gap.

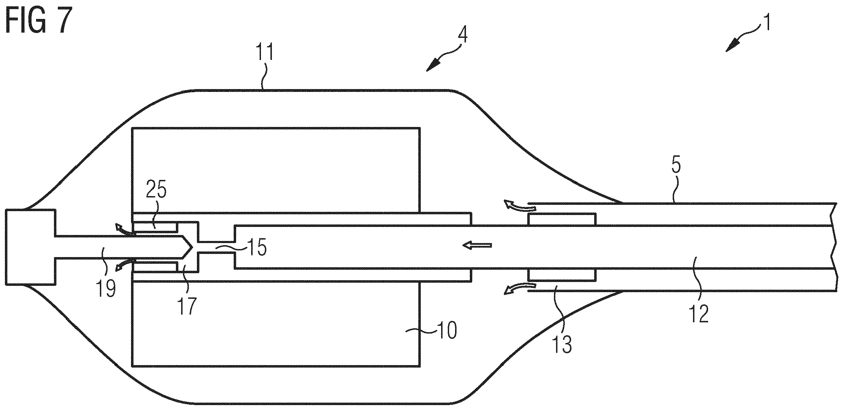

Fig. 7 shows the intravascular blood pump 1 with its catheter 5 and its pump section 4. In this embodiment, the intravascular blood pump 1 comprises a proximal bearing 13 inside the distal end of the catheter 5. Purge fluid may now flow through the catheter 5 and exit the proximal bearing 13 through its bearing gap. Some of the purge fluid also flows through the drive shaft 12 into the rotor 10.

The bearing gap of the proximal bearing is preferably between 1 µm and 10 µm, more preferably between 2 µm and 8 µm.

From the drive shaft 12 inside the rotor, the purge fluid flows through the fluid line 15 into the recess 17 of the rotor 10. Arranged inside the recess 17 is a distal bearing sleeve 25 of the rotor 10. The inner surface of the distal bearing sleeve 25 and the outer surface of the pin 19 form the bearing surfaces of the distal bearing 14. The purge fluid leaves the rotor 10 via the bearing gap between the distal bearing sleeve 25 and the pin 19.

The distal bearing sleeve 25 has an inner diameter of preferably between 0.3 mm and 1.5 mm, more preferably between 0.5 mm and 1.2 mm and most preferably between 0.7 mm and 0.9 mm. The outer diameter of the distal bearing sleeve 25 is preferably between 0.5 mm and 1.7 mm, more preferably between 0.7 mm and 1.4 mm and most preferably between 0.9 mm and 1.1 mm. The bearing gap between the pin 19 and the distal bearing sleeve 25 is preferably between 1 µm and 10 µm, more preferably between 2 µm and 8 µm.

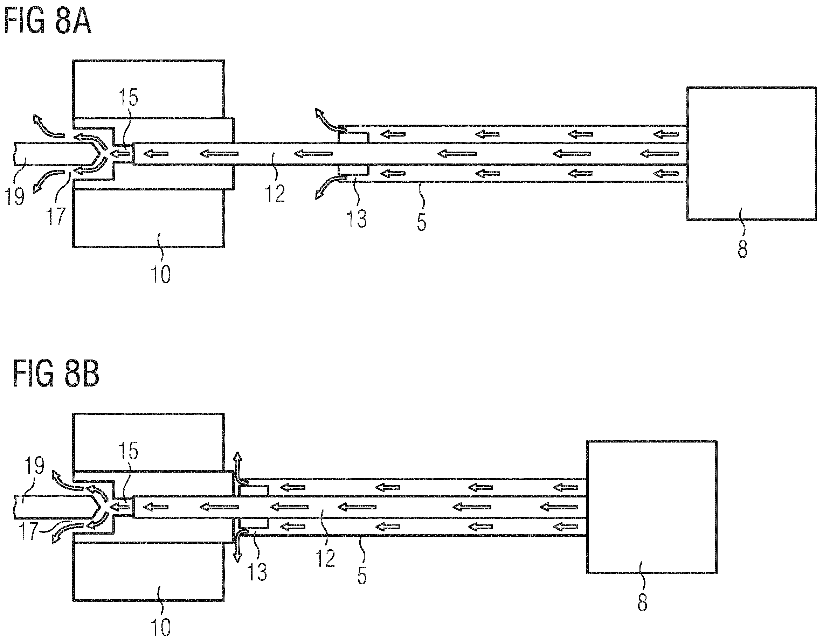

Fig. 8A shows schematically the purge fluid path inside the intravascular blood pump. Inside the housing of the motor 8, the purge fluid is supplied into the catheter 5 and into the drive shaft 12. At the proximal bearing 13, purge fluid leaves the catheter 5 through the bearing gap to reduce friction and cool the proximal bearing 13. A portion of the purge fluid does not leave the catheter 5 through the bearing gap but flows through the drive shaft 12 into the rotor 10. In some embodiments, the drive shaft 12 may comprise a cover such that the purge fluid may flow from the catheter 5 to the rotor 10 without leaking from the drive shaft 12 between the distal end of the catheter 5 and the proximal end of the rotor 10. Inside the rotor 10, the purge fluid continues to flow through the fluid line 15 and then into the recess 17 at the distal end of the rotor 10. In alternative embodiments, the drive shaft 12 may continue up to or into the recess 17 such that the purge fluid flows into the recess 17 directly from the drive shaft 12. From there, the purge fluid flows through the bearing gap of the distal bearing 14 between the pin 19 and the adjacent surface of the rotor 10.

Fig. 8B shows an embodiment of the blood pump similar to Fig. 8A. In Fig. 8B, the proximal bearing 13 is closer to the rotor 10 than in Fig. 8A and is separated from the rotor 10 only by a small gap. Through said gap, purge fluid may escape as shown by arrows.

## Claims

1. An intravascular blood pump (1), comprising a pumping device and a catheter (5), wherein the pumping device comprises:
a drive shaft (12);
a rotor (10) located at a distal end of the drive shaft (5);
a housing (11), in which the rotor (10) is housed, and
a distal bearing (14) for rotatably supporting a distal end of the rotor (10),
**characterized in that** the distal bearing (14) comprises a static support member (18),
which protrudes into or up against the distal end of the rotor (10).

2. The intravascular blood pump according to claim 1, wherein the intravascular blood pump (1) is designed as an expandable pump, wherein a cannula (16) is provided around a portion of the drive shaft (12), which lies in the vicinity of the rotor (10), the housing (11) and the rotor (10) being configured to be transferred at least in part into the cannula (12), wherein during such transfer an expandable section of the housing (11) and the rotor (10) are compressed at least along a radial direction extending transversely to a longitudinal direction, from an expanded state to a compressed state.

3. The intravascular blood pump according to claim 2, wherein in the expanded state of the housing (11), the static support member (18) protrudes up against the distal end of the rotor (10) with a force equal to or less than 1.8 N.

4. The intravascular blood pump according to claim 2 or 3, wherein in the compressed state of the housing (11), the static support member (18) is located at a distance from the rotor (10).

5. The intravascular blood pump according to any one of the claims 2 to 4, wherein the rotor (10) comprises a nose (21) at a distal end thereof, which protrudes into the static support member (18) when the housing (11) is in its expanded state.

6. The intravascular blood pump according to any one of claims 1, 2 or 4, wherein the rotor (10) comprises an axial stop for the static support member (18).

7. The intravascular blood pump according to any one of claims 1 to 6, comprising a fluid line (15) inside the distal end of the drive shaft (12), which is arranged to guide a purge fluid to the distal bearing.

8. The intravascular blood pump according to claim 7, wherein the rotor (10) comprises a hollow section as a part of the fluid line (15), wherein the intravascular blood pump (1) is arranged to guide a purge fluid through the hollow section of the rotor to the distal bearing (14).

9. The intravascular blood pump according to claim 7 or 8, wherein the distal bearing (14) is arranged such that, when purge fluid is guided through the fluid line (15) with sufficient pressure, at least a portion of the purge fluid exits between the static support member (18) and the distal end of the rotor (10), into which or up against which the static support member (18) protrudes.

10. The intravascular blood pump according to any one of claims 1 to 9, wherein a distal end of the static support member (18) is mounted at a distal end of the housing (11).

11. The intravascular blood pump according to any one of claims 1 to 10, wherein the static support member (18) comprises a pin (19) extending from distally to proximally, the pin (19) protruding into the distal end of the rotor (10).

12. The intravascular blood pump according to claim 11, wherein a bearing gap between an outside of the pin (19) and a bearing surface opposing the outside of the pin (19) has a width in the range from 1 µm to 10 µm, preferably from 2 µm to 8 µm.

13. The intravascular blood pump according to any one of claims 11 to 12, wherein a length of the pin (19) by which the pin protrudes into the distal end of the rotor (10) during an operational state of the intravascular blood pump (1) is in the range from 0.5 mm to 8 mm, preferably from 1 mm to 5 mm, most preferably from 1.5 mm to 2.5 mm.

14. The intravascular blood pump according to any one of claims 11 to 13 with claim 2, wherein the pin (19) has a sufficient length to remain within the distal end of the rotor (10) when the housing (11) and the rotor (10) are in the compressed state.

15. The intravascular blood pump according to any one of claims 11 to 14, wherein the pin (19) is made of a material which comprises at least one of the following materials: MP35N, 35NLT, Nitinol, stainless steel and ceramics.

16. The intravascular blood pump according to any one of claims 1, 2, 4 and 6 to 15, wherein an inner diameter at the distal end of the rotor (10), into which the static support member (18) protrudes, is in the range from 0.3 mm to 1.5 mm, more preferably from 0.5 mm to 1.2 mm and most preferably from 0.7 mm to 0.9 mm.

17. A method of using the intravascular blood pump (1) according to any one of claims 1 to 16 in a patient such that blood flow in the patient is supported by means of the intravascular blood pump (1).
